**Europäisches Patentamt**

**European Patent Office**

**Office européen des brevets**

(11) Numéro de publication: **0 019 607**
**A1**

(12) ## DEMANDE DE BREVET EUROPEEN

(21) Numéro de dépôt: **80870027.2**

(22) Date de dépôt: **08.05.80**

(51) Int. Cl.³: **C 25 F 3/00**
**A 45 D 27/46, A 61 L 2/02**

(30) Priorité: **14.05.79 LU 81255**

(43) Date de publication de la demande:
**26.11.80 Bulletin 80/24**

(84) Etats Contractants Désignés:
**AT BE CH DE FR GB IT LI NL SE**

(71) Demandeur: **Louys, Jean-Marie**
**134, Boulevard de la Sauvenière, Bte 42**
**B-4000 Liège(BE)**

(72) Inventeur: **Louys, Jean-Marie**
**134, Boulevard de la Sauvenière, Bte 42**
**B-4000 Liège(BE)**

(74) Mandataire: **van Malderen, Michel et al,**
**p.a. FREYLINGER & ASSOCIES 145, Boulevard de la**
**Sauvenière(Bte 31)**
**B-4000 Liége(BE)**

(54) **Procédé d'aiguisage ou d'affûtage de lames.**

(57) Fixée au manche du rasoir (10), la lame est introduite dans une solution comportant un électrolyte et de préférence un antiseptique et/ou un agent tensio-actif. La solution est ou a été soumise à l'effet de deux électrodes (6,7) reliées à une source de courant continu de faible voltage.
Application aux lames de rasoir.

Fig.1.

EP 0 019 607 A1

- 1 -

## Procédé d'aiguisage ou d'affûtage de lames.

La présente invention concerne un procédé d'aiguisage ou d'affûtage de lames, en particulier de lames de rasoir à jeter.

On sait que de nombreuses recherches ont été effectuées en vue de développer des lames de rasoir dites à jeter.

On entend par lames de rasoir à jeter des lames interchangeables pouvant être montées dans un rasoir conçu à cet effet ou encore des lames montées à demeure sur un rasoir à jeter ou bien encore des lames montées sur un support (bloc) de petites dimensions pouvant être inséré sur le rasoir ou détaché de celui-ci.

Ces lames peuvent être soit simples soit multiples et sont généralement réalisées en un acier spécial permettant un affûtage très précis offrant des qualités de coupe remarquables.

Dans certains cas, ces lames sont montées par deux sur un support en vue de réaliser un meilleur rasage (lames jumelées).

On constate généralement après un certain nombre d'utilisations successives que les lames sont émoussées et qu'elles doivent être remplacées.

Le but visé par la présente invention est de fournir un procédé permettant d'aiguiser ou d'affûter une lame ayant subi un affûtage mécanique préalable ou de réaiguiser ou réaffûter une lame émoussée.

Le procédé de l'invention peut être répété plusieurs fois sur une même lame et permet de prolonger la durée d'utilisation de celle-ci.

Bien qu'il soit fait référence dans la description qui suit à des lames de rasoir, il doit être bien entendu que l'invention est également applicable à d'autres dispositifs ou outils à lames en vue d'un aiguisage ou d'un affûtage.

- 2 -

Le procédé selon l'invention est caractérisé en ce que la lame à aiguiser ou affûter est introduite dans une solution comportant un électrolyte et qui est ou qui a été soumise à l'effet de deux électrodes reliées à une source de courant continu de faible voltage.

Il n'est pas nécessaire pour obtenir un effet satisfaisant sur une lame de rasoir que celle-ci soit maintenue entre les deux électrodes pendant la mise sous tension de celle-ci. Il suffit dans la plupart des cas que la solution ait été soumise pendant quelque temps à l'action du courant continu à l'intervention des électrodes et qu'ensuite le rasoir soit laissé dans cette solution pendant un temps de l'ordre de quelques minutes, au moins.

Pour des lames fortement usées il est cependant recommandable que la lame soit introduite dans la solution et y soit laissée un certain temps pendant que les électrodes sont reliées à la source de courant continu.

L'électrolyte peut être constitué par les sels contenus habituellement dans l'eau alimentaire mais il est avantageux, en vue de constituer la solution, d'utiliser de l'eau alimentaire contenant un agent antiseptique et/ou un agent tensioactif, les adjuvants étant compatibles avec les exigences cosmétiques.

Dans le cas d'une application à des lames de rasoir, on s'est aperçu qu'en réalisant un bain contenant les constituants: chloroxylénol 4,9 g; huile de pin aromatique; isopropanol, savon, laurylsulfate de sodium, ainsi que de l'eau pour amener à 100 g, dilué à raison de 5 ml pour 500 ml d'eau, on peut obtenir un effet convenable lorsqu'on soumet les lames, disposées entre deux électrodes de préférence inattaquables pendant une durée de l'ordre de 1 à 15 minutes à une tension continue de l'ordre de 4,5 volts aux électrodes.

Bien que le Demandeur n'entende se limiter à aucune explication théorique du phénomène qui se produit dans les

- 3 -

conditions indiquées, il est supposé que le premier des constituants (chloroxylénol) agisant comme oxydant, en présence des autres constituants exerçant une fonction d'électrolyte, réalise sous l'effet d'électrolyse un traitement de surface sur le tranchant de la lame.

Il est probable aussi qu'il se produit un effet de polarisation de la lame qui agit sur le poil de la barbe et facilite sa coupe.

Une caractéristique importante de l'invention est l'absence d'irritation et de rougeurs que l'utilisateur observe après rasage. L'utilisation d'un antiseptique et d'une meilleure qualité de coupe de la lame pourrait expliquer ce phénomène.

En répétant plusieurs fois l'opération indiquée, il est possible d'obtenir d'excellents résultats permettant de réutiliser de nombreuses fois la lame après traitement.

Pour l'exécution du procédé, il est avantageux de disposer le rasoir dans un récipient de forme appropriée contenant deux électrodes reliées à une source de courant continu, qui est constituée par exemple par une pile. Avantageusement, des interrupteurs sont prévus dans le circuit pour fermer celui-ci sous l'effet de la fermeture du couvercle du récipient, le circuit pouvant être ouvert après un certain temps de fonctionnement par tout moyen approprié, par exemple un dispositif de temporisation.

Selon une forme d'exécution plus simple, il est prévu un interrupteur qui ferme le circuit pendant le temps du rasage et qui est actionné à la fin du rasage pour réouvrir le circuit et éviter d'user inutilement les piles.

L'invention sera décrite plus en détail à l'aide d'un schéma représentant un dispositif adéquat pour la mise en oeuvre du procédé et un exemple de mise en pratique donné à titre d'illustration mais sans aucun caractère limitatif.

- 4 -

L'essai a été réalisé sur des lames de rasoir jumelées du type Gillette G2 et a permis d'effectuer 50 à 100 rasages consécutifs tout en gardant un parfait état de coupe, alors que pour la plupart des utilisateurs la durée d'usage normale varie de 1 à 10 rasages successifs.

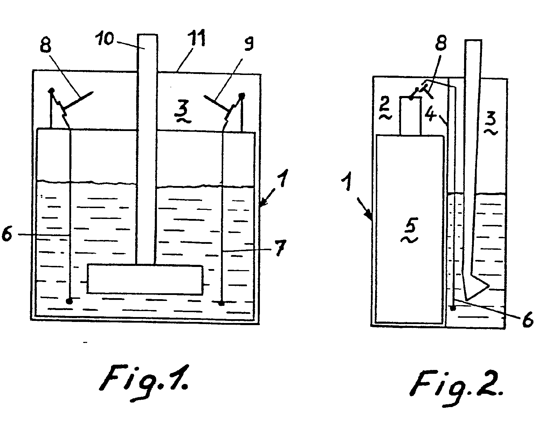

Dans les figures 1 et 2 on a représenté respectivement une vue de face et une vue de profil d'un dispositif adéquat.

Ce dispositif est constitué par un récipient portant le repère général 1, de forme généralement quelconque, qui sera de préférence cependant parallélipipédique pour des raisons d'encombrement. Ce récipient est constitué d'une première chambre 2 et d'une seconde chambre 3 séparées par une paroi 4. Dans la chambre 2 est disposée une source de courant continue 5, constituée par exemple par une pile de 4,5 volts dont les deux pôles sont reliés respectivement à des électrodes 6 et 7 disposées dans le compartiment 3. Entre les bornes respectives de la pile 5 et les électrodes 6 et 7, on a interposé des interrupteurs 8 et 9.

L'écartement entre les électrodes 6 et 7 est suffisant pour qu'on puisse introduire entre elles le rasoir 10 portant les lames jumelées. Le récipient peut être fermé par un couvercle 11.

Les électrodes sont réalisées en un matériau inaltérable résistant à la solution utilisée.

On introduit dans la chambre, 75 ml d'eau alimentaire et 5 ml de DETTOL de la composition : chloroxylénol 4,9 g - Ol. pini aromaticum - isopropanolum - sapo - Natrii laurylsulfas - saccharum ustum - Aqua ad 100 g.

On relève initialement les lectures suivantes.

échelle 0,3 lecture          0,07 volts

échelle 100 $\mu$ lecture       20 $\mu$ ampères

après 10 minutes

échelle 0,3 lecture          0,13 volts

échelle 100 $\mu$ lecture       40 $\mu$ ampères.

On laisse le rasoir et on établit le courant électrique dans la solution en fermant les interrupteurs. La durée du traitement est de 6 minutes. Les valeurs relevées sont :

| | |
|---|---|
| échelle 1 lecture | 0,45 volts fond du récipient |
| échelle 300 mA lecture | fond d'échelle |
| | |
| échelle 1 lecture | 0,6 volts en surface |
| échelle 300 mA lecture | fond d'échelle. |

Après 6 minutes, en laissant le rasoir dans la solution, le courant électrique est interrompu et on relève à ce moment les lectures suivantes :

| | |
|---|---|
| échelle 1 V.lecture | 0,10 volts fond du récipient |
| échelle 100 $\mu$ lecture | 34 $\mu$ ampère |
| | |
| échelle 1 V.lecture | 0,25 volts en surface |
| échelle 100 $\mu$ lecture | 92 $\mu$ ampère en surface. |

Pour conserver un affûtage ou aiguisage adéquat de la lame, le traitement peut se répéter après 4 ou 5 rasages.

Les mesures électriques effectuées l'ont été avec des appareillages peu précis et sont sujettes à correction, mais elles permettent cependant des lectures comparatives.

Le phénomène qui se produit n'a pas été élucidé par le Demandeur et pourrait également s'apparenter à un effet d'électrisation pouvant attirer le poil de la barbe, en plus de l'effet d'affûtage.

Cependant des essais effectués sur d'autres ustensiles et outils destinés à d'autres usages que le rasage permettent également d'observer une meilleure qualité d'aiguisage.

Il convient en particulier de noter que le traitement tel que décrit peut être appliqué également à des lames de rasoir neuves en vue de constituer un traitement final de celles-ci. En effet, on observe que dans les fabrications, des variations de qualité des lames se produisent régulièrement, quels que soient les soins mis à leur affûtage. L'invention pourrait donc avantageusement s'appliquer en vue

- 6 -

d'uniformiser la qualité des lames neuves avant leur mise sur le marché.

Bien qu'on ait décrit des formes d'exécution particulièrement préférées de l'invention, il doit être bien entendu que de nombreuses variantes et adaptations sont possibles tout en restant dans le cadre de l'invention. C'est ainsi qu'en particulier, la nature des constituants du bain n'est nullement limitative et qu'il est possible de substituer les constituants décrits par d'autres constituants exerçant un effet équivalent, aussi bien du point de vue du caractère oxydant que du point de vue du caractère détergent, électrolytique ou antiseptique.

Revendications

1. Procédé d'aiguisage ou d'affûtage de lames, en particulier de lames de rasoir caractérisé en ce que la lame à affûter ou à aiguiser est introduite dans une solution comportant un électrolyte et qui est ou qui a été soumise à l'effet de deux électrodes reliées à une source de courant continu de faible voltage comportant au moins l'un des constituants : chloroxylénol et/ou détergent anionique.

2. Procédé selon la revendication 1 caractérisé en ce que la solution est constituée d'eau, d'un électrolyte et d'un agent antiseptique.

3. Procédé selon la revendication 1 ou 2 caractérisé en ce que la solution contient un agent tensio-actif.

4. Procédé selon la revendication 1 caractérisé en ce qu'on utilise un bain contenant les constituants : chloroxylénol 4,9 g; huile de pin aromatique; isopropanol, savon, laurylsulfate de sodium, ainsi que de l'eau pour amener à 100 g, diluée à raison de 5 ml pour 500 ml d'eau en soumettant les lames disposées entre deux électrodes inattaquables pendant une durée de l'ordre de 1 à 15 minutes à une tension continue de 4,5 volts.

5. Procédé selon l'une quelconque des revendications 1 à 4, caractérisé en ce qu'on dispose le rasoir dans un récipient contenant deux électrodes reliées par un circuit muni d'un interrupteur à une source de courant continu constituée par une pile.

6. Procédé selon la revendication 5 caractérisé en ce qu' un interrupteur est prévu dans le circuit, pour fermer celui-ci sous l'effet de la fermeture du couvercle du récipient, le circuit étant ouvert après un certain temps de fonctionnement par tout moyen approprié, tel qu'un dispositif de temporisation.

Fig.1.

Fig.2.

Office européen
des brevets

**RAPPORT DE RECHERCHE EUROPEENNE**

Numéro de la demande

EP 80 87 0027

| Catégorie | DOCUMENTS CONSIDERES COMME PERTINENTS | | CLASSEMENT DE LA DEMANDE (Int. Cl. 3) |
|---|---|---|---|
| | Citation du document avec indication, en cas de besoin, des parties pertinentes | Revendication concernée | |
| | DE - A - 1 521 095 (WILKINSON SWORD) <br><br> * En entier * <br> -- | 1,3 | C 25 F 3/00 <br> A 45 D 27/46 <br> A 61 L 2/02 |
| | US - A - 3 352 630 (FISCHER) <br><br> * Colonne 3, lignes 55-60; revendications 1-10 * <br> -- | 1 | |
| | US - A - 2 667 456 (YOUNG) <br><br> * En entier * <br> -- | 1 | |
| | GB - A - 2 006 834 (MORGANS) <br><br> * Page 1; page 2, revendications 1-40 * <br> -- | 1 | |
| | US - A - 3 334 035 (DEWS) <br><br> * Revendications 1-3 * <br> -- | 1 | |
| A | US - A - 4 027 387 (KELLIS) | | |
| A | US - A - 3 492 178 (WESTLING) | | |
| A | GB - A - 607 406 (LLOYD-THOMAS) | | |
| A | FR - A - 808 932 (QUAITSCH) | | |
| A | CH - A - 198 002 (HOFLER) | | |
| A | DE - A - 1 565 925 (WILKINSON SWORD) | | |
| A | DE - C - 450 052 (MAYR) | | |
| | ./.. | | |

**DOMAINES TECHNIQUES RECHERCHES (Int. Cl. 3)**

C 25 F  3/00
       3/02
       3/06
       3/16
       3/22
       3/24
       7/00
A 61 L  2/02
       2/16
       2/18
A 45 D  27/46
B 23 P  1/04
B 26 B  21/54

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 20.08.1980 | VAN LEEUWEN |

OEB Form 1503.1 06.78

Numéro de la demande

EP 80 87 0027
-2-

Office européen
des brevets

**RAPPORT DE RECHERCHE EUROPEENNE**

| | DOCUMENTS CONSIDERES COMME PERTINENTS | | CLASSEMENT DE LA DEMANDE (Int. Cl. 3) |
|---|---|---|---|
| Catégorie | Citation du document avec indication, en cas de besoin, des parties pertinentes | Revendication concernée | |
| A | W.B. HUGO: "Ihibitation and destruction of the microbial cell", page 81, Academic Press, 1971 LONDRES (GB) | | |
| | | | DOMAINES TECHNIQUES RECHERCHES (Int. Cl. 3) |

OEB Form 1503.2 06.78